**Europäisches Patentamt**

**European Patent Office**

**Office européen des brevets**

(11) Veröffentlichungsnummer : **0 373 442 B1**

(12) **EUROPÄISCHE PATENTSCHRIFT**

(45) Veröffentlichungstag der Patentschrift :
07.04.93 Patentblatt 93/14

(21) Anmeldenummer : 89122185.5

(22) Anmeldetag : 01.12.89

(51) Int. Cl.⁵ : **C08F 220/18,** C08F 226/10,
A61K 7/06, A61K 7/11,
// (C08F220/18, 226:10),
(C08F226/10, 220:18)

(54) **Copolymerisate auf Basis von tert.-Butylacrylat und/oder tert.-Butylmethacrylat.**

(30) Priorität : **15.12.88 DE 3842183**

(43) Veröffentlichungstag der Anmeldung :
**20.06.90 Patentblatt 90/25**

(45) Bekanntmachung des Hinweises auf die
Patenterteilung :
**07.04.93 Patentblatt 93/14**

(84) Benannte Vertragsstaaten :
**DE ES FR GB IT NL**

(56) Entgegenhaltungen :
**DE-A- 1 911 306**
**DE-A- 2 216 260**
**DE-A- 3 627 969**
**DE-A- 3 627 970**

(73) Patentinhaber : **BASF Aktiengesellschaft**
**Carl-Bosch-Strasse 38**
**W-6700 Ludwigshafen (DE)**

(72) Erfinder : **Potthoff-Karl, Birgit, Dr.**
**Grundelbachstrasse 112 e**
**W-6940 Weinheim (DE)**
Erfinder : **Sperling-Vietmeier, Karin, Dr.**
**Im Kirchenstueck 12**
**W-6730 Neustadt (DE)**
Erfinder : **Sanner, Axel, Dr.**
**Lorscher Ring 2 c**
**W-6710 Frankenthal (DE)**

EP 0 373 442 B1

## Beschreibung

Die vorliegende Erfindung betrifft Copolymerisate auf der Basis von tert.-Butylacrylat und/oder tert.-Butylmethylacrylat mit einem K-Wert von 10 bis 50, erhältlich durch radikalische Polymerisation von

A) 20 bis 90 Gew.% tert.-Butylacrylat und/oder tert.-Butylmethacrylat,

B) 10 bis 60 Gew.% N-Vinylpyrrolidon und

C) 0 bis 30 Gew.% eines $C_1$-$C_{20}$-Alkylacrylats, $C_1$-$C_{20}$-Alkylmethacrylats, $C_2$-$C_4$-Hydroxyalkylacrylats, $C_2$-$C_4$-Hydroxyalkylmethacrylats oder Vinylacetat oder Gemischen dieser Monomeren.

Außerdem betrifft die Erfindung die Verwendung dieser Copolymerisate als Filmbildner in Haarfestigungsmitteln und Mitteln zum Oberflächenschutz von flächigen oder räumlichen Gebilden.

Für die Haarkosmetik werden in zunehmendem Maße Sprayzubereitungen mit Kohlenwasserstoffen anstelle von halogenierten Kohlenwasserstoffen als Treibmittel eingesetzt. Man verwendet hierfür als Filmbildner häufig Polymere mit sauren oder basischen Gruppen. So werden beispielsweise in der DE-A 36 27 969 Copolymerisate aus N-Vinylpyrrolidon, N-Mono- oder N,N-Dialkylacrylamiden und Alkyl- oder Hydroxyalkylestern der Acryl- oder Methacrylsäure und/oder Acryl- oder Methacrylsäure und in der DE-A 36 27 970 Terpolymerisate aus N-Vinylpyrrolidon, tert.-Butylacrylat oder -methacrylat und Acryl- oder Methylacrylsäure beschrieben.

Die carboxylgruppenhaltigen Polymerisate müssen zur besseren Verträglichkeit mit den unpolaren Kohlenwasserstoffen der Sprayzubereitungen, d.h. zur besseren Löslichkeit in ihnen, mit Aminen, zum Beispiel mit 2-Amino-2-methylpropanol, neutralisiert werden. Dies kann eventuell Geruchsprobleme bei der Haarbehandlung mit sich bringen. Die Kohlenwasserstoffverträglichkeit und auch die haarfestigende Wirkung dieser Produkte liegen zwar schon auf hohem Niveau, jedoch sind Verbesserungen dieser Eigenschaften noch wünschenswert.

Ähnliche Überlegungen wie für Haarsprayzubereitungen gelten für Sprayzubereitungen zum Oberflächenschutz von flächigen oder räumlichen Gebilden. Das Aufbringen dieser auch als Firnisse bezeichneten Schutzfilme erfolgt immer häufiger mit Hilfe von Sprayzubereitungen, die Kohlenwasserstoffe als Treibmittel enthalten. Daher bedarf es auch hier noch der Verbesserung der Kohlenwasserstoffverträglichkeit der Polymerisate.

Die BE-A 899 078 betrifft allgemein Copolymerisate aus 40 bis 90 mol% N-Vinylpyrrolidon und 10 bis 60 mol% eines oder mehrerer $C_2$-$C_8$-Alkylester der Acryl- oder Methacrylsäure. In dieser Veröffentlichung sind jedoch nur Copolymere mit Acrylsäure- und Methacrylsäurealkylestern beschrieben, in denen der Alkylrest unverzweigt ist. Diese Copolymere dienen als Bindemittel für medizinisches Verbandmaterial und ähnliche Erzeugnisse.

Aufgabe der vorliegenden Erfindung war es, einen Filmbildner für die Haarkosmetik und den Oberflächenschutz von flächigen oder räumlichen Gebilden bereitzustellen, der sich durch eine gute Verträglichkeit mit unpolaren Treibmitteln auf der Basis von Kohlenwasserstoffen auszeichnet und eine gleichbleibend gute haarfestigende Wirkung bzw. Schutzfilmwirkung zeigt.

Demgemäß wurden die eingangs definierten Copolymerisate gefunden.

Die erfindungsgemäßen Copolymerisate werden aus 20 bis 90 Gew.%, vorzugsweise 35 bis 90 Gew.% tert.-Butylacrylat und/oder -methacrylat als Komponente A und 10 bis 60 Gew.%, vorzugsweise 10 bis 35 Gew.%, N-Vinylpyrroliden als Komponente B hergestellt. Besonders gute Anwendungseigenschaften werden bei einer Zusammensetzung von 50 bis 90 Gew.% tert.-Butylacrylat und/oder -methacrylat und 10 bis 30 Gew.% N-Vinylpyrrolidon erreicht.

Außerdem können die erfindungsgemäßen Copolymerisate bis zu 30 Gew.%, vorzugsweise bis zu 20 Gew.%, aus einem $C_1$-$C_{20}$-Alkylacrylat oder -methacrylat, beispielsweise Methyl-, Ethyl-, n-Propyl-, Isopropyl-, n-Butyl-, Isobutyl-, sec.-Butyl-, n-Pentyl-, n-Hexyl-, 2-Ethylhexyl-, n-Octyl-, n-Nonyl-, Isononyl-, n-Decyl-, n-Dodecyl-, n-Tridecyl-, Isotridecyl-, Myristyl-, Cetyl-, Stearyl- oder Eicosylacrylat bzw. -methacrylat, aus einem $C_2$-$C_4$-Hydroxyalkylacrylat oder -methacrylat, beispielsweise 2-Hydroxyethyl-, 2-Hydroxypropyl-, 3-Hydroxypropyl-, 3-Hydroxybutyl- oder 4-Hydroxybutylacrylat bzw. -methacrylat, aus Vinylacetat oder aus Gemischen dieser Monomeren als Komponente C mitaufgebaut sein. Für die Komponente C kommen vor allem $C_1$-$C_4$-Alkylacrylate oder -methacrylate, Hydroxypropylacrylate oder -methacrylate und insbesondere Vinylacetat in Betracht.

Die erfindungsgemäßen Copolymerisate werden durch radikalische Copolymerisation von tert.-Butylacrylat und/oder -methacrylat mit N-Vinylpyrrolidon und gegebenenfalls einem oder mehreren Monomeren C, vorzugsweise durch Lösungspolymerisation in einem organischen Lösungsmittel, in der Regel einem Alkohol, hergestellt. Man arbeitet hier üblicherweise bei Temperaturen von 60 bis 130°C, wobei die Umsetzung bei Normaldruck oder unter Eigendruck durchgeführt werden kann.

Als Initiatoren für die radikalisch ablaufende Polymerisationsreaktion können die üblichen Peroxo- oder Azoverbindungen, beispielsweise Dibenzoylperoxid, tert.-Butylperpivalat, tert.-Butylper-2-ethylhexanoat, Di-tert.-butylperoxid, tert.-Butylhydroperoxid, 2,5-Dimethyl-2,5-di(tert.-butylperoxy)hexan oder Azo-bis-isobuty-

2

ronitril, zweckmäßigerweise in Mengen von 0,1 bis 2 Gew.%, bezogen auf das Gewicht der Monomeren, eingesetzt werden. Man wählt die Mengen an Monomeren und Lösungsmittel zweckmäßigerweise so, daß man 30 bis 80 gew.%ige Lösungen der Copolymerisate erhält.

Die erfindungsgemäßen Copolymerisate weisen K-Werte von 10 bis 50, vorzugsweise 15 bis 35, auf. Der jeweils gewünschte K-Wert läßt sich in an sich bekannter Weise durch Wahl der Polymerisationsbedingungen, in erster Linie der Polymerisationsdauer und der Initiatorkonzentration, einstellen. Die K-Werte werden nach Fikentscher, Cellulosechemie, Bd. 13, S. 58 bis 64 (1932) bei 25°C in 1,0 gew.%iger ethanolischer Lösung gemessen und stellen ein Maß für das Molgewicht dar.

Die erfindungsgemäßen Copolymerisate haben üblicherweise Glasübergangs-temperaturen zwischen 50 und 130°C, insbesondere zwischen 60 und 100°C.

Die erfindungsgemäßen Copolymerisate finden hauptsächlich als Filmbildner in Haarfestigungsmitteln Verwendung, zum Beispiel in Haargelen, Haarschäumen und vor allem als Frisurenfestiger in Haarsprays. Außerdem können sie, vorzugsweise in Form von Sprayzubereitungen, zum Oberflächenschutz von flächigen oder räumlichen Gebilden, beispielsweise Zeichnungen, Karten, Drucken, Fotos oder Bildern, durch Aufbringen eines transparenten Schutzfilmes benutzt werden.

Besonders bevorzugt werden für diese beiden Anwendungsbereiche Sprayzubereitungen, die die folgenden Bestandteile enthalten:

- 0,1 bis 20 Gew.%, vorzugsweise 0,5 bis 10 Gew.%, insbesondere 2 bis 5 Gew.% des erfindungsgemäßen Copolymerisats
- 10 bis 95 Gew.%, vorzugsweise 20 bis 60 Gew.%, insbesondere 30 bis 50 Gew.% eines üblichen Lösungsmittels wie vor allem Ethanol und Isopropanol und daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische
- 5 bis 90 Gew.%, vorzugsweise 30 bis 80 Gew.%, insbesondere 45 bis 70 Gew.% eines üblichen Treibmittels wie Propan, n-Butan, Isobutan, 2,2-Dimethylbutan, Isopentan, Dimethylether, Fluortrichlormethan, Dichlordifluormethan oder Dichlortetrafluorethan oder deren Gemische. Als Treibmittel (Treibgas) kommen von den genannten Verbindungen vor allem die Kohlenwasserstoffe und insbesondere Propan und n-Butan zur Anwendung. Gegebenenfalls werden einer oder mehrere der genannten Fluorchlorkohlenwasserstoffe in Treibmittelmischungen mitverwendet, jedoch nur in geringen Mengen, etwa bis zu 20 Gew.%, bezogen auf die Treibmittelmischung.

Außerdem können diese Sprayzubereitungen noch geringe Mengen an Parfümölen, beispielsweise 0,1 bis 5,0 Gew.%, enthalten.

Eine Standard-Sprayformulierung weist beispielsweise die folgende Zusammensetzung auf:

3 Gew.% des erfindungsgemäßen Copolymerisates

30 Gew.% Ethanol

67 Gew.% Propan/n-Butan im Gewichtsverhältnis von 40:60.

Ein überwiegender Teil des Ethanols kann durch ein anderes Lösungsmittel, beispielsweise einen Kohlenwasserstoff wie n-Pentan oder n-Hexan, ersetzt werden, ohne daß sich die haarfestigende Wirkung verschlechtert. Als Treibmittel kann auch n-Butan alleine verwendet werden.

Die erfindungsgemäßen Copolymerisate zeichnen sich durch ihre hohe Verträglichkeit mit unpolaren Treibmitteln in Sprayzubereitungen, insbesondere mit Kohlenwasserstoffen wie Propan oder n-Butan oder ihren Gemischen, aus. In der Regel erreicht man mit ihnen Verträglichkeitswerte zwischen 75 und 90 Gew.%, wogegen die genannten Polymerisate des Standes der Technik Werte aufweisen, die in den meisten Fällen unter 70 Gew.% liegen. Gleichzeitig zeigen die erfindungsgemäßen Copolymerisate eine außergewöhnlich gute haarfestigende Wirkung, ersichtlich an den hohen Werten für die Curl-Retention, die im Bereich von 80 bis 95 liegen.

Beispiel 1

Copolymerisat aus tert.-Butylacrylat und N-Vinylpyrrolidon

Eine Lösung von 35 g tert.-Butylacrylat, 15 g N-Vinylpyrrolidon und 0,5 g tert.-Butylperpivalat in 275 g Ethanol wurde auf 75°C erwärmt. Nach dem Anspringen der Polymerisation, erkennbar an einer Viskositätserhöhung, wurden gleichzeitig eine Mischung aus 315 g tert.-Butylacrylat, 135 g N-Vinylpyrrolidon und 100 g Ethanol und eine Lösung von 4,8 g tert.-Butylperpivalat in 120 g Ethanol im Laufe von 6 Stunden zugegeben, wobei die Temperatur bei schwachem Sieden auf 77 bis 80°C gehalten wurde. Anschließend wurden 3,0 g 2,5-Dimethyl-2,5-di(tert.-butylperoxy)hexan auf einmal zugegeben, wonach der Reaktionskessel druckfest verschlossen, auf 130°C aufgeheizt und 3 Stunden bei dieser Temperatur gehalten wurde.

Der Polymerisatgehalt der erhaltenen Lösung betrug 48,5 Gew.%. Das Copolymerisat hatte einen K-Wert von 19,3 (gemessen in 1,0 gew.%iger ethanolischer Lösung bei 25°C) und eine Glasübergangstemperatur von 75°C.

Beispiel 2

Copolymerisat aus tert.-Butylmethacrylat und N-Vinylpyrrolidon

Analog Beispiel 1 wurde aus tert.-Butylmethacrylat und N-Vinylpyrrolidon eine ca. 50 gew.%ige ethanolische Copolymerisatlösung hergestellt. Das Copolymerisat hatte einen K-Wert von 21,1 (gemessen in 1,0 gew.%iger ethanolischer Lösung bei 25°C) und eine Glasübergangstemperatur von 87°C.

Beispiel 3

Copolymerisat aus tert.-Butylacrylat, N-Vinylpyrrolidon und Vinylacetat

Eine Mischung aus 60 g tert.-Butylacrylat, 30 g N-Vinylpyrrolidon, 10 g Vinylacetat, 0,7 g tert.-Butylperpivalat und 60 g Ethanol wurde auf 75°C erwärmt. Nach dem Anspringen der Polymerisation, erkennbar an einer Viskositätserhöhung, wurden gleichzeitig eine Mischung aus 540 g tert.-Butylacrylat, 270 g N-Vinylpyrrolidon, 90 g Vinylacetat und 550 g Ethanol und eine Lösung von 6,0 g tert.-Butylperpivalat in 90 g Ethanol im Laufe von 6 Stunden zugegeben, wobei die Temperatur bei schwachem Sieden auf 77 bis 80°C gehalten wurde. Anschließend wurde eine Lösung von 3,0 g 2,5-Dimethyl-2,5-di(tert.-butylperoxy)hexan in 400 g Ethanol zugegeben, wonach der Reaktionskessel druckfest verschlossen, auf 130°C aufgeheizt und 3 Stunden bei dieser Temperatur gehalten wurde.

Der Polymerisatgehalt der erhaltenen Lösung betrug 47,7 Gew.%. Das Terpolymerisat hatte einen K-Wert von 22,9 (gemessen in 1,0 gew.%iger ethanolischer Lösung bei 25°C) und eine Glasübergangstemperatur von 79°C.

Beispiele 4 bis 12

Die Beispiele 4 bis 12 (siehe Tabelle) betreffen Copolymerisate aus tert.-Butylacrylat und N-Vinylpyrrolidon oder tert.-Butylacrylat, N-Vinylpyrrolidon und Vinylacetat jeweils unterschiedlicher Zusammensetzung. Die Produkte 4 bis 9 wurden analog Beispiel 1 und die Produkte 10 bis 12 analog Beispiel 3 hergestellt.

Eigenschaften der Copolymerisate

Die folgende Tabelle zeigt die Werte für die Zusammensetzung, die Kohlenwasserstoffverträglichkeit und die haarfestigende Wirkung der Copolymerisate aus den Beispielen 1 bis 12. Zum Vergleich sind als Beispiel A und B die entsprechenden Werte für zwei Copolymere gemäß der DE-A 36 27 970 angegeben.

Tabelle: Zusammensetzung, Kohlenwasserstoffverträglichkeit und Curl-Retention von Haarsprayformulierungen

| Beispiel | Zusammensetzung [Gew.%] | | | | | Kohlenwasser-stoffverträglich-keit mit Propan/n-Butan (40:60) [Gew.%] | Curl-Retention [%] |
|---|---|---|---|---|---|---|---|
| | tBA | tBMA | VP | VAc | AS | | |
| 1 | 70 | | 30 | | | 83 | 93 |
| 2 | | 70 | 30 | | | 81 | 89 |
| 3 | 60 | | 30 | 10 | | 80 | 85 |
| 4 | 40 | | 60 | | | 76 | 78 |
| 5 | 50 | | 50 | | | 77 | 93 |
| 6 | 60 | | 40 | | | 81 | 90 |
| 7 | 75 | | 25 | | | 84 | 90 |
| 8 | 85 | | 15 | | | 88 | 83 |
| 9 | 90 | | 10 | | | 89 | 89 |
| 10 | 45 | | 40 | 15 | | 72 | 86 |
| 11 | 70 | | 25 | 5 | | 82 | 86 |
| 12 | 80 | | 15 | 5 | | 83 | 89 |
| Vergleichs-beispiele:* | | | | | | | |
| A | 50 | | 40 | | 10 | 71 | 56 |
| B | | 50 | 40 | | 10 | 64 | 75 |

tBA: tert.-Butylacrylat, tBMA: tert.-Butylmethacrylat, VP: Vinylpyrrolidon
VAc: Vinylacetat, AS: Acrylsäure
* gemäß Beispiel 2 und 3 der DE-A 36 27 970

Der Wert für die Kohlenwasserstoffverträglichkeit mit einer Propan/n-Butan-Mischung im Gewichtsverhältnis von 40:60 gibt an, wieviel Gew.% dieses Treibgasgemisches eine Sprayzubereitung, die neben Ethanol als Lösungsmittel 3 Gew.% des Copolymeren aufweist, maximal enthalten darf, ohne daß bei 0°C eine Trübung auftritt.

Die Curl-Retention ist ein Maß für die haarfestigende Wirkung. Sie wird im Modellversuch an Haarlocken gemessen, die durch eine übliche Wasserwelle an ca. 15 cm langen Haaren erzeugt und mit der jeweiligen Sprayzubereitung aus 10 cm Entfernung 4 sec lang besprüht worden sind. Nach einer Verweilzeit von 5 Stunden der aufgehängten Locken in einer Klimakammer bei 25°C und 90 % relativer Luftfeuchtigkeit wird die relative Verformung (Aufweitung) der Locken, bezogen auf ihre ursprüngliche Form, bestimmt. Ein hoher Wert bedeutet eine hohe Festigungswirkung, d.h. bei 100 % wäre die ursprüngliche Form vollständig erhalten.

Die Curl-Retention wurde jeweils mit der folgenden Standard-Sprayformulierung bestimmt:

3 Gew.% des erfindungsgemäßen Copolymerisats
30 Gew.% Ethanol
67 Gew.% Propan/n-Butan (40:60)

Beispiel 13

Sprayzubereitung zum Oberflächenschutz von flächigen Gebilden

Mit einer Sprayformulierung aus

3 Gew.% Copolymerisat aus Beispiel 8 (85 Gew.% tert.-Butylacrylat, 15 Gew.% N-Vinylpyrrolidon)
20 Gew.% Ethanol
30 Gew.% n-Hexan
47 Gew.% n-Butan

wurde bedrucktes Papier besprüht. Man erhielt einen transparenten, beständigen und schmutzabweisenden Schutzfilm.


**Patentansprüche**

**Patentansprüche für folgende Vertragsstaaten : DE, FR, GB, IT, NL**

1. Copolymerisate auf der Basis von tert.-Butylacrylat und/oder tert.-Butylmethacrylat mit einem K-Wert von 10 bis 50, erhältlich durch radikalische Polymerisation von
   A) 20 bis 90 Gew.% tert.-Butylacrylat und/oder tert.-Butylmethacrylat,
   B) 10 bis 60 Gew.% N-Vinylpyrrolidon und
   C) 0 bis 30 Gew.% eines $C_1$-$C_{20}$-Alkylacrylats, $C_1$-$C_{20}$-Alkylmethacrylats, $C_2$-$C_4$-Hydroxyalkylacrylats, $C_2$-$C_4$-Hydroxyalkylmethacrylats oder Vinylacetat oder Gemischen dieser Monomeren.

2. Copolymerisate nach Anspruch 1, erhältlich durch radikalische Polymerisation von
   A) 35 bis 90 Gew.% tert.-Butylacrylat und/oder tert.-Butylmethacrylat,
   B) 10 bis 35 Gew.% N-Vinylpyrrolidon und
   C) 0 bis 30 Gew.% der Komponente C.

3. Verfahren zur Herstellung von Copolymerisaten gemäß Anspruch 1 durch radikalische Polymerisation von
   A) 20 bis 90 Gew.% tert.-Butylacrylat und/oder tert.-Butylmethacrylat,
   B) 10 bis 60 Gew.% N-Vinylpyrrolidon und
   C) 0 bis 30 Gew.% der Komponente C.

4. Verfahren zur Herstellung von Copolymerisaten gemäß Anspruch 2 durch radikalische Polymerisation von
   A) 35 bis 90 Gew.% tert.-Butylacrylat und/oder tert.-Butylmethacrylat,
   B) 10 bis 35 Gew.% N-Vinylpyrrolidon und
   C) 0 bis 30 Gew.% der Komponente C.

5. Haarfestigungsmittel, enthaltend als Filmbildner ein Copolymerisat gemäß Anspruch 1 oder 2.

6. Mittel zum Oberflächenschutz von flächigen oder räumlichen Gebilden, enthaltend als Filmbildner ein Copolymerisat gemäß Anspruch 1 oder 2.

7. Haarsprayzubereitungen, enthaltend neben hierbei üblichen Lösungsmitteln und Treibmitteln 0,1 bis 20 Gew.% eines Copolymerisats gemäß Anspruch 1 oder 2.

8. Sprayzubereitungen zum Oberflächenschutz von flächigen oder räumlichen Gebilden, enthaltend neben hierbei üblichen Lösungsmitteln und Treibmitteln 0,1 bis 20 Gew.% eines Copolymerisats gemäß Anspruch 1 oder 2.

9. Verwendung der Copolymerisate gemäß Anspruch 1 oder 2 als Filmbildner in Haarfestigungsmitteln.

10. Verwendung der Copolymerisate gemäß Anspruch 1 oder 2 als Filmbildner in Mitteln zum Oberflächenschutz von flächigen oder räumlichen Gebilden.

**Patentansprüche für folgenden Vertragsstaat : ES**

1. Verfahren zur Herstellung von Copolymerisaten mit einem K-wert von 10 bis 50 durch radikalische Polymerisation von
   A) 20 bis 90 Gew.% tert.-Butylacrylat und/oder tert.-Butylmethacrylat,
   B) 10 bis 60 Gew.% N-Vinylpyrrolidon und
   C) 0 bis 30 Gew.% eines $C_1$-$C_{20}$-Alkylacrylats, $C_1$-$C_{20}$-Alkylmethacrylats, $C_2$-$C_4$-Hydroxyalkylacrylats, $C_2$-$C_4$-Hydroxyalkylmethacrylats oder Vinylacetat oder Gemischen dieser Monomeren.

2. Verfahren zur Herstellung von Copolymerisaten nach Anspruch 1 durch radikalische Polymerisation von
A) 35 bis 90 Gew.% tert.-Butylacrylat und/oder tert.-Butylmethacrylat,
B) 10 bis 35 Gew.% N-Vinylpyrrolidon und
C) 0 bis 30 Gew.% der Komponente C.

3. Verfahren zur Herstellung von Haarfestigungsmitteln, dadurch gekennzeichnet, daß man Copolymerisate gemäß Anspruch 1 oder 2 mit den hierbei üblichen Bestandteilen mischt.

4. Verfahren zur Herstellung von Mitteln zum Oberflächenschutz von flächigen oder räumlichen Gebilden, dadurch gekennzeichnet, daß man Copolymerisate gemäß Anspruch 1 oder 2 mit den hierbei üblichen Bestandteilen mischt.

5. Verfahren zur Herstellung von Haarsprayzubereitungen, dadurch gekennzeichnet, daß man 0,1 bis 20 Gew.% eines Copolymerisates gemäß Anspruch 1 oder 2, bezogen auf die Gesamtmenge der Zubereitung, mit den hierbei üblichen Lösungsmitteln und Treibmitteln mischt.

6. Verfahren zur Herstellung von Sprayzubereitungen zum Oberflächenschutz von flächigen oder räumlichen Gebilden, dadurch gekennzeichnet, daß man 0,1 bis 20 Gew.% eines Copolymerisates gemäß Anspruch 1 oder 2, bezogen auf die Gesamtmenge der Zubereitung, mit den hierbei üblichen Lösungsmitteln und Treibmitteln mischt.

## Claims

### Claims for the following Contracting States : DE, FR, GB, IT, NL

1. A copolymer based on tert-butyl acrylate or tert-butyl methacrylate and having a K value of from 10 to 50, obtainable by free radical polymerization of
A) from 20 to 90 % by weight of tert-butyl acrylate or tert-butyl methacrylate,
B) from 10 to 60 % by weight of N-vinylpyrrolidone and
C) from 0 to 30 % by weight of a $C_1$-$C_{20}$-alkyl acrylate, a $C_1$-$C_{20}$-alkyl methacrylate, a $C_2$-$C_4$-hydroxyalkyl acrylate, a $C_2$-$C_4$-hydroxyalkyl methacrylate or vinyl acetate or a mixture thereof.

2. A copolymer as claimed in claim 1 obtainable by free radical polymerization of
A) from 35 to 90 % by weight of tert-butyl acrylate or tert-butyl methacrylate,
B) from 10 to 35 % by weight of N-vinylpyrrolidone and
C) from 0 to 30 % by weight of component C.

3. A process for preparing a copolymer as claimed in claim 1 by free radical polymerization of
A) from 20 to 90 % by weight of tert-butyl acrylate or tert-butyl methacrylate,
B) from 10 to 60 % by weight of N-vinylpyrrolidone and
C) from 0 to 30 % by weight of component C.

4. A process for preparing a copolymer as claimed in claim 2 by free radical polymerization of
A) from 35 to 90 % by weight of tert-butyl acrylate or tert-butyl methacrylate,
B) from 10 to 35 % by weight of N-vinylpyrrolidone and
C) from 0 to 30 % by weight of component C.

5. A hair setting composition containing a copolymer as claimed in claim 1 or 2 as film former.

6. A composition for the surface protection of two-or three-dimensional structures containing a copolymer as claimed in claim 1 or 2 as film former.

7. A hair spray formulation containing besides customary solvents and propellants from 0.1 to 20 % by weight of a copolymer as claimed in claim 1 or 2.

8. A spray formulation for the surface protection of two- or three-dimensional structures containing besides customary solvents and propellants from 0.1 to 20 % by weight of copolymer as claimed in claim 1 or 2.

9. The use of a copolymer as claimed in claim 1 or 2 as a film former in hair setting compositions.

10. The use of a copolymer as claimed in claim 1 or 2 as a film former in compositions for the surface protection of two- or three-dimensional structures.

**Claims for the following Contracting State : ES**

1. A process for preparing a copolymer having a K value of from 10 to 50 by free radical polymerization of
A) from 20 to 90 % by weight of tert-butyl acrylate or tert-butyl methacrylate,
B) from 10 to 60 % by weight of N-vinylpyrrolidone and
C) from 0 to 30 % by weight of a $C_1$-$C_{20}$-alkyl acrylate, a $C_1$-$C_{20}$-alkyl methacrylate, a $C_2$-$C_4$-hydroxyalkyl acrylate, a $C_2$-$C_4$-hydroxyalkyl methacrylate or vinyl acetate or a mixture thereof.

2. A process for preparing a copolymer as claimed in claim 1 by free radical polymerization of
A) from 35 to 90 % by weight of tert-butyl acrylate or tert-butyl methacrylate,
B) from 10 to 35 % by weight of N-vinylpyrrolidone and
C) from 0 to 30 % by weight of component C.

3. A process for preparing a hair setting composition, which comprises mixing a copolymer as prepared in claim 1 or 2 with the customary ingredients.

4. A process for preparing a composition for the surface protection of two- or three-dimensional structures, which comprises mixing a copolymer as prepared in claim 1 or 2 with the customary ingredients.

5. A process for preparing a hair spray formulation, which comprises mixing from 0.1 to 20% by weight of a copolymer as prepared in claim 1 or 2, based on the total amount of the formulation, with the customary solvents and propellants.

6. A process for preparing a spray formulation for the surface protection of two- or three-dimensional structures, which comprises mixing from 0.1 to 20% by weight of a copolymer as prepared in claim 1 or 2, based on the total amount of the formulation, with the customary solvents and propellants.

**Revendications**

**Revendications pour les Etats contractants suivants : DE, FR, GB, IT, NL**

1. Copolymères à base d'acrylate de butyle tertiaire et/ou de méthacrylate de butyle tertiaire, ayant une valeur K de 10 à 50, pouvant être obtenus par polymérisation radicalaire de
A) 20 à 90% en poids d'acrylate de butyle tertiaire et/ou de méthacrylate de butyle tertiaire,
B) 10 à 60% en poids de N-vinylpyrrolidone et
C) 0 à 30% en poids d'un acrylate d'alkyle en $C_1$-$C_{20}$, d'un méthacrylate d'alkyle en $C_1$-$C_{20}$, d'un acrylate d'hydroxyalkyle en $C_2$-$C_4$, d'un méthacrylate d'hydroxyalkyle en $C_2$-$C_4$, d'acétate de vinyle ou de mélanges de ces monomères.

2. Copolymères selon la revendication 1, pouvant être obtenus par polymérisation radicalaire de
A) 35 à 90% en poids d'acrylate de butyle tertiaire et/ou de méthacrylate de butyle tertiaire,
B) 10 à 35% en poids de N-vinylpyrrolidone et
C) 0 à 30% en poids du composant C.

3. Procédé de préparation de copolymères selon la revendication 1 par polymérisation radicalaire de
A) 20 à 90% en poids d'acrylate de butyle tertiaire et/ou de méthacrylate de butyle tertiaire,
B) 10 à 60% en poids de N-vinylpyrrolidone et
C) 0 à 30% en poids du composant C.

4. Procédé de préparation de copolymères selon la revendication 2 par polymérisation radicalaire de
A) 35 à 90% en poids d'acrylate de butyle tertiaire et/ou de méthacrylate de butyle tertiaire,
B) 10 à 35% en poids de N-vinylpyrrolidone et
C) 0 à 30% en poids du composant C.

5. Fixateur pour cheveux, contenant un copolymère selon la revendication 1 ou 2 comme filmogène.

6. Produit pour la protection de la surface d'objets bi- ou tridimentionnels, contenant un copolymère selon la revendication 1 ou 2 comme filmogène.

7. Préparations de laque à pulvériser sur les cheveux, contenant de 0,1 à 20% en poids d'un copolymère selon la revendication 1 ou 2, en plus de solvants et d'agents propulseurs usuels pour de tels produits.

8. Préparations à pulvériser pour la protection de la surface d'objets bi- ou tridimensionnels, contenant de 0,1 à 20% en poids d'un copolymère selon la revendication 1 ou 2, en plus de solvants et d'agents propulseurs usuels pour de tels produits.

9. Utilisation des copolymères selon la revendication 1 ou 2 comme filmogènes dans des fixateurs pour cheveux.

10. Utilisation des copolymères selon la revendication 1 ou 2 comme filmogènes dans des produits pour la protection de la surface d'objets bi- ou tridimensionnels.

**Revendications pour l'Etat contractant suivant : ES**

1. Procédé de préparation de copolymères ayant une valeur K de 10 à 50, par polymérisation radicalaire de
A) 20 à 90% en poids d'acrylate de butyle tertiaire et/ou de méthacrylate de butyle tertiaire,
B) 10 à 60% en poids de N-vinylpyrrolidone et
C) 0 à 30% en poids d'un acrylate d'alkyle en $C_1$-$C_{20}$, d'un méthacrylate d'alkyle en $C_1$-$C_{20}$, d'un acrylate d'hydroxyalkyle en $C_2$-$C_4$, d'un méthacrylate d'hydroxyalkyle en $C_2$-$C_4$, d'acétate de vinyle ou de mélanges de ces monomères.

2. Procédé de préparation de copolymères selon la revendication 1, par polymérisation radicalaire de
A) 35 à 90% en poids d'acrylate de butyle tertiaire et/ou de méthacrylate de butyle tertiaire,
B) 10 à 35% en poids de N-vinylpyrrolidone et
C) 0 à 30% en poids du composant C.

3. Procédé de préparation de fixateurs pour cheveux, caractérisé en ce qu'on mélange des copolymères selon la revendication 1 ou 2 avec les constituants usuels pour de tels produits.

4. Procédé de préparation de produits pour la protection de la surface d'objets bi- ou tridimensionnels, caractérisé en ce qu'on mélange des copolymères selon la revendication 1 ou 2 avec les constituants usuels pour de tels produits.

5. Procédé de fabrication de préparations à pulvériser sur les cheveux, caractérisé en ce qu'on mélange de 0,1 à 20% en poids d'un copolymère selon la revendication 1 ou 2, par rapport à la quantité totale de la préparation, avec les solvants et agents propulseurs usuels pour de telles préparations.

6. Procédé de fabrication de préparations à pulvériser pour la protection de la surface d'objets bi- ou tridimensionnels, caractérisé en ce qu'on mélange de 0,1 à 20% en poids d'un copolymère selon la revendication 1 ou 2, par rapport à la quantité totale de la préparation, avec les solvants et agents propulseurs usuels pour de telles préparations.